# EUROPEAN PATENT APPLICATION

(11) **EP 3 563 673 A1**
(43) Date of publication of application: **06.11.2019**
(21) Application number: 18747967.0
(22) Date of filing: 31.01.2018
(51) Int. Cl.: A01H 1/00, C12N 5/04

(54) **METHOD FOR INTRODUCING SUBSTANCE INTO PLANT**

(30) Priority: 31.01.2017 JP 2017015371
(71) Applicant: Japan Tobacco Inc., Tokyo 105-8422 (JP); Riken, Wako-shi, Saitama 351-0198 (JP); Tokyo Metropolitan University, Tokyo 163-0926 (JP)
(72) Inventor: KATO, Norio, Iwata-shi Shizuoka 438-0802 (JP); ICHIKAWA, Masako, Iwata-shi Shizuoka 438-0802 (JP); OKAMOTO, Takashi, Hachioji-shi Tokyo 192-0397 (JP); KOISO, Narumi, Hachioji-shi Tokyo 192-0397 (JP); KIBA, Takatoshi, Wako-shi Saitama 351-0198 (JP); TODA, Erika, Wako-shi Saitama 351-0198 (JP)
(74) Representative: Isarpatent
(86) International application number: PCT/JP2018/004103
(87) International publication number: WO 2018/143480

(57) **Abstract**

The present invention relates to a method for introducing a substance into a plant. The method of the present invention comprises introducing a substance into a plant germ cell with incomplete cell wall formation.

## Description

### TECHNICAL FIELD

The present invention relates to a method for introducing a substance into a plant.

### BACKGROUND ART

Transgenic technologies in plants, particularly, in monocotyledonous plants, have been widely adopted rapidly, since methods using Agrobacterium for rice and maize have been developed in the 1990's. Various transformation methods have been developed so far. However, it is known that the efficiency of transformation greatly differs among species and varieties, since many of such methods need to go through dedifferentiation and redifferentiation of plant tissues. For certain species and varieties, the efficiency of transformation is low, and transformed plants with reproducibility cannot be obtained. For example, in B73, which is a very important strain for maize breeding, general transformation methods which efficiently yield transformed plants have not yet been developed.

Further, it is becoming possible to efficiently practice genome editing in recent years. However, practical use thereof is also hindered since the point that the ease of tissue culture differs depending on crop species and variety greatly affects the efficiency of genome editing.

Meanwhile, artificial fertilization in which a sperm cell and an egg cell isolated from a plant are artificially fused (in-vitro fertilization) has been attempted in the 1990's, and a plant has been successfully produced. Non-Patent Literature (NPL) 1 discloses a method of producing a fertilized egg cell (in-vitro fertilized egg) by electrofusion of an egg cell and a sperm cell of maize and culturing them into a plant. In NPL 1, a highly concentrated mixture of plant tissue-degrading enzymes is used for separating an egg cell. Further, NPL 2 discloses a method of producing a fertilized egg cell by electrofusion of male and female gametes of rice and culturing it into a plant. Artificial fertilization of a plant (in-vitro fertilization system) is composed of three steps of: isolating gamete cells (an egg cell and a sperm cell) from a flower before pollination, fusing the isolated cells (fertilization), and culturing the fused cell (fertilized egg). Typical successful examples include maize (NPL 1) and rice (NPL 2), and some examples of wheat and tobacco have been also reported. Generally, an egg cell is obtained by cutting the ovary before pollination or disassembling the ovary or ovules treated with plant tissue-degrading enzymes such as cellulase and pectinase under a microscope, and a sperm cell is obtained by bursting pollen in a suitable osmotic solution. Thereafter, the male and female gametes are transferred to a fusing droplet using a glass capillary. Three types of methods for fusing gamete cells, electrical fusion (NPLs 1 and 2), fusion using calcium ions (NPL 3), and polyethylene glycol fusion (NPLs 4 and 5) have been reported. However, only fertilized egg cells produced by electrical fusion have been reported to grow into embryos so as to be regenerated into plants (NPLs 1 and 2). These prior art literatures indicate that plants can be induced from a fertilized egg cell obtained by artificially fusing male and female gametes. However, gene introduction into a fertilized egg cell or transformation thereof are not mentioned at all, and it has been totally unknown whether or not transformation can be performed using an in-vitro fertilized egg cell.

For species such as maize (NPL 6), rice (NPL 7), wheat (NPL 8), barley (NPLs 9 and 11), and tobacco (NPL 10), examples in which a fertilized egg cell is collected and cultured from the ovary or ovules after fertilization to produce a plant are known. NPLs 6 and 11, which relate to maize and barley, disclose introduction of DNA into a fertilized egg cell by the microinjection method. However, it has not been reported that a plant transformation method of a fertilized egg cell by the microinjection method is practically used. Further, gene introduction by other methods has not been found at all.

The microinjection method enables gene introduction into a cell having a cell wall, and there is no particular need to remove the cell wall of a plant cell as an introduction target by a treatment with plant tissue-degrading enzymes or the like. However, there is a disadvantage that only one cell can be handled in one introduction operation, and it is not suitable for gene introduction experiments of middle to large scale using many plant cells. Further, since complicated operations under a microscope and skilled techniques are required for the microinjection, its' practical use has been difficult. Examples of the method for introducing a gene into a cell include the polyethylene glycol method (polyethylene glycol: PEG method), the peptide method (NPL 12), the electroporation method, and the Agrobacterium method, other than the microinjection method. The electroporation method, the peptide method, and the PEG method, particularly, the PEG method have advantages that their approaches are simple as compared with the microinjection method and many cells can be handled at one time. However, a plant cell has a cell wall, and therefore a tissue and a cell are generally treated with plant tissue-degrading enzymes such as cellulase, pectinase, protease, and hemicellulase particularly in the PEG method or the electroporation method, to dissolve the cell wall, thereby protoplastizing the cell. From these facts, materials from which a large amount of protoplasts can be obtained, such as leaves, cultured cells, and calli have been used as targets in the aforementioned methods (NPLs 13 and 14).

Meanwhile, unlike leaves, cultured cells, and calluses, production and isolation of fertilized egg cells are time-consuming and cannot yield a large amount of protoplasts. Further, an egg cell or a sperm cell immediately before fertilization does not start differentiation unless they are fused by an electrical treatment, addition of a calcium solution, or the like. In such a fertilized egg cell obtained in an in-vitro fertilization system, some damage may have possibly occurred in the cell due to the operation for artificial fertilization such as fusing as described above. Further, it has been unknown how to remove a cell wall from a fertilized egg cell, while maintaining the cell activity which enables the cell to continuously divide after the removal of the cell wall so as to grow into a plant. Under such circumstances, an egg cell, a sperm cell, and a fertilized egg cell have been considered as being unsuitable as a target into which a gene is introduced. Therefore, it has not been reported that a gene is introduced by a method such as the PEG method to achieve cell division.

### CITATION LIST

### PATENT LITERATURE

PTL 1: Japanese Patent Laid-Open No. 2016-63785 (PTL 1)

### NON PATENT LITERATURE

NPL 1: Kranz E. and Loerz H., (1993), Plant Cell 5:739-746.
NPL 2: Uchiumi, T. et al., (2007), Planta 226:581-589.
NPL 3: Faure, J.E. et el., (1994), Science 263:1598-1600.
NPL 4: Sun, M.X. et al., (1995), Acta Bot Sin 36:489-493.
NPL 5: Tian, H.Q. and Russell, S.D. (1997), Plant Cell Rep 16: 657-661.
NPL 6: Leduc, N. et al., (1996), Developmental Biology 177: 190-203.
NPL 7: Zhang, J. et al., (1999), Plant Cell Reports 19: 128-132.
NPL 8: Kumlehn, J. et al., (1997), Plant Cell Reports 16: 663-667.
NPL 9: Holm, P.B. et al., (1994), The Plant Cell 6: 531-543.
NPL 10: Yuchi, H.E. et al., (2004), Chinese Science Bulletin 49: 810-814.
NPL 11: Holm, P.B. et al., (2000), Transgenic Research 9: 21-32.
NPL 12: Laksmanan, M. et.al., (2012), Biomacromolecules 14, 10-16.
NPL 13: Yoo, S.D. et al. (2007), Nature Protocol 2: 1565-1572.
NPL 14: Zhai, Z. et al. (2009), Plant Physiol. 149: 642-652.
NPL 15: Kranz, E. et al., (1995), Plant Journal 8: 9-23.
NPL 16: Toda, E. et al., (2016), Plant Physiology 171: 206-214.
NPL 17: Koiso, N. et al., (2017), Plant Direct 1: e00010
NPL 18: Hiei, Y. and Komari, T., (2008). Nature Protocols 3: 824-834.

### SUMMARY OF INVENTION

### TECHNICAL PROBLEM

It is an object of the present invention to provide a method for introducing a substance into a plant, and a plant into which a substance is introduced by the method of the present invention.

A fertilized egg cell is a cell that originally possesses the ability to grow into a plant and therefore is expected not to be affected by the difference in culture efficiency between species or varieties. Treatments such as transformation and genome editing can be performed on a wider variety of species or crops by introducing a substance into a fertilized egg cell that is a zygote, or an egg cell or a sperm cell that is a gamete as a target than in the current situation. As a result of diligent studies, the inventors found a method that enables substance introduction into a fertilized egg cell without a treatment with plant tissue-degrading enzymes. Further, they have found that substance introduction into a fertilized egg cell, division induction, and transformation are enabled by combining methods for culturing an isolated fertilized egg cell without a treatment with plant tissue-degrading enzymes, thereby achieving the present invention.

### SOLUTION TO PROBLEM

The present invention includes, though not limited to, the following embodiments.

### [Embodiment 1]

A method for introducing a substance into a plant, comprising introducing a substance into a plant germ cell with incomplete cell wall formation.

### [Embodiment 2]

The method according to embodiment 1, wherein the plant germ cell isolated is not treated with a plant tissue-degrading enzyme before introduction of the substance.

### [Embodiment 3]

The method according to embodiment 1 or 2, wherein the rate of cell wall formation is 65% or less.

### [Embodiment 4]

The method according to embodiment 1, wherein the plant germ cell is a fertilized egg cell.

### [Embodiment 5]

The method according to any one of embodiments 1 to 4, comprising:
obtaining a fertilized egg cell by
(1-i) fusing an egg cell and a sperm cell of a plant to produce a fertilized egg cell, or
(1-ii) isolating a fertilized egg cell of a plant from a tissue containing the fertilized egg cell; and
(2) introducing the substance into the fertilized egg cell obtained.

### [Embodiment 6]

The method according to embodiment 5, wherein the egg cell and the sperm cell are fused in (1-i) by electrofusion.

### [Embodiment 7]

The method according to embodiment 5 or 6, wherein step (2) of introducing the substance is performed within 120 minutes after the fertilized egg cell is obtained.

### [Embodiment 8]

The method according to embodiment 5 or 6, wherein step (2) of introducing the substance is performed within 60 minutes after the fertilized egg cell is obtained.

### [Embodiment 9]

The method according to embodiment 5, wherein the egg cell and the sperm cell are fused in (1-i) by natural fertilization.

### [Embodiment 10]

The method according to embodiment 5 or 9, wherein step (2) of introducing the substance is performed within 360 minutes after the fertilized egg cell is obtained.

### [Embodiment 11]

The method according to embodiment 5 or 9, wherein step (2) of introducing the substance is performed within 240 minutes after the fertilized egg cell is obtained.

### [Embodiment 12]

The method according to any one of embodiments 1 to 4, comprising
(1) introducing the substance into either or both of the egg cell and the sperm cell of the plant; and
(2) fusing the egg cell and the sperm cell that have undergone step (1) to produce a fertilized egg cell.

### [Embodiment 13]

The method according to embodiment 12, wherein the egg cell and the sperm cell are fused in (1) by electrofusion or natural fertilization.

### [Embodiment 14]

The method according to any one of embodiments 1 to 13, wherein the substance is introduced using a PEG method or an electroporation method.

### [Embodiment 15]

The method according to any one of embodiments 1 to 14, wherein the plant is a monocotyledonous plant.

### [Embodiment 16]

The method according to embodiment 15, wherein the plant is selected from the group consisting of maize, wheat, barley, rice, and sorghum.

### [Embodiment 17]

A substance-introduced plant obtained by the method according to any one of embodiments 1 to 16.

### ADVANTAGEOUS EFFECTS OF INVENTION

Conventionally, transformation by the PEG method has been considered as being unsuitable for a fertilized egg cell produced in vitro by an electrofusion treatment or the like. In the present invention, substance introduction, transformation, and culture can be performed by utilizing "a plant germ cell with incomplete cell wall formation" without treating a cell with plant tissue-degrading enzymes. The present invention enables transformants of plants, which have been conventionally difficult to transform due to the difficulty of culture or the like and thus to which useful traits could not be given, to be stably obtained with good reproducibility.

### BRIEF DESCRIPTION OF DRAWINGS

[Fig. 1] Fig. 1 includes optical micrographs of an egg cell (upper row) and a sperm cell (lower row) isolated from a rice flower.
[Fig. 2] Fig. 2 includes optical micrographs showing the process of fusing the egg cell and the sperm cell in Fig. 1 and a fertilized egg cell produced (in-vitro fertilized egg cell).
[Fig. 3] Fig. 3 includes fluorescence micrographs of a fertilized egg cell of rice which has started to divide after introduction of GFP nucleic acids by the PEG method. Fig. 3A includes the results of fluorescence observation (left) and bright field observation (right) 24 hours after fusion of gametes. Fig. 3B includes the results of fluorescence observation (left) and bright field observation (right) 2 days after fusion of gametes.
[Fig. 4] Fig. 4 includes fluorescence and optical micrographs of a cell mass derived from a fertilized egg cell of rice into which GFP nucleic acids have been introduced. 18 days after fusion of gametes, fluorescence observation (left) and bright field observation (right) were performed. A cell mass in which fluorescence from the fertilized egg was continuously observed (A), and a cell mass in which fluorescence was not detected (B) were observed.
[Fig. 5 ]Fig. 5 is an optical micrograph of a shoot generated from a cell mass derived from a fertilized egg cell of rice into which GFP nucleic acids have been introduced.
[Fig. 6] Fig. 6 is a seedling derived from the shoot generated from the cell mass of Fig. 5.
[Fig. 7] Fig. 7 is a graph showing the amount of plasmids in a fertilized rice egg 1 day after GFP plasmid introduction. Those into which nucleic acids are introduced are shown as PEG treatment +, and those in which GFP fluorescence is observed by a fluorescence microscope are shown as GFP signal +.
[Fig. 8] Fig. 8 includes fluorescence micrographs (upper row), optical micrographs (lower row), and their merges (middle row) of a fertilized maize egg into which GFP nucleic acids are introduced (in vitro) and a cell mass derived from the fertilized egg cell.

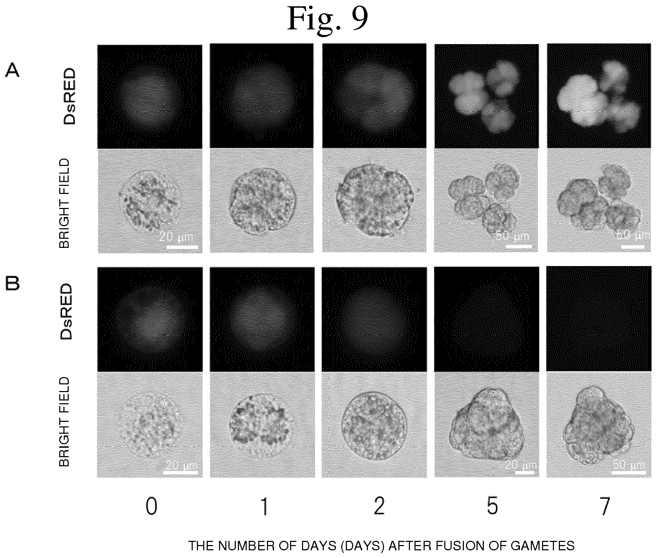

[Fig. 9] Fig. 9 includes fluorescence micrographs (upper row) and optical micrographs (lower row) of a fertilized egg derived from DsRed2-transformed rice and a cell mass derived from a fertilized egg cell of DsRed2-transformed rice into which genome-editing nucleic acids are introduced. A cell obtained by culturing a fertilized egg cell derived from DsRed2-transformed rice produced by fusion of gametes (without introduction of genome-editing nucleic acids) is shown as A, and a cell obtained by introducing genome-editing nucleic acids (plasmid DNA) into a fertilized egg cell of DsRed2-transformed rice produced by fusing gametes, followed by culture, is shown as B. In the fertilized egg of DsRed2-transformed rice into which genome-editing nucleic acids are introduced, it was shown that the fluorescence of DsRed2 was disappeared.
[Fig. 10] Fig. 10 includes fluorescence micrographs (left and center) and an optical micrograph (right) of a rice egg cell into which two types of nucleic acids, GFP and DsRed2, are introduced. GFP fluorescence observation (left), RFP fluorescence observation (center), and bright field observation (right) were performed 24 hours after the introduction of nucleic acids.

### DESCRIPTION OF EMBODIMENTS

The present invention relates to a method for introducing a substance into a plant.

The method of the present invention comprises introducing a substance into a plant germ cell with incomplete cell wall formation.

According to one embodiment, the method comprises:
obtaining a fertilized egg cell by
(1-i) fusing an egg cell and a sperm cell of a plant to produce a fertilized egg cell, or
(1-ii) isolating a fertilized egg cell of a plant from a tissue containing the fertilized egg cell; and
(2) introducing the substance into the fertilized egg cell obtained.

According to one embodiment, the method comprises:
(1) introducing the substance into either or both of the egg cell and the sperm cell of the plant; and
(2) fusing the egg cell and the sperm cell that have undergone step (1) to produce a fertilized egg cell.

### Plant

The types of the plant are not specifically limited. Any one of dicotyledonous plants and monocotyledonous plants may be employed, and preferably, monocotyledonous plants are employed. Further preferably, Gramineae plants, more preferably, maize, wheat, barley, rice, sorghum, rye, and the like, and most preferably, maize, wheat, and rice are employed.

The method of the present invention can be particularly used for, though not limited to, "difficult-to-culture" plants or varieties. The term "difficult-to-culture" means that culture is difficult, specifically, for example, culture of a cell isolated from a plant is difficult, or callus formation by a treatment such as dedifferentiation, or redifferentiation from a callus into a plant is difficult.

Generally, culture of monocotyledonous plants is more difficult than that of dicotyledonous plants, but the "difficult-to-culture" plants, for example, include soybeans, common beans, capsicums, and the like. The difficult-to-culture varieties mean varieties culture of which is more difficult than that of general research varieties (such as maize A188) of the same species. Examples thereof include maize B73, maize elite varieties derived from B73, wheat elite varieties (such as AC Barrie and TAM), barley varieties other than GoldenPromise and Igri, and sorghum varieties other than 296B, C401, SA281, P898012, Pioneer 8505, and Tx430.

### Plant germ cell

The method of the present invention introduces a substance into a germ cell of a plant.

The germ cell is a cell playing a role in transmitting genetic information to the next generation through reproduction and is a cell other than somatic cells among cells constituting a multicellular organism. In this description, the "germ cell" includes a gamete for sexual reproduction including an egg cell (also referred to as "egg" or "ovum") and a sperm cell (also referred to as "sperm"), and a zygote (also referred to as "conjugant") which is a cell produced as a result of mating gametes, and includes a fertilized egg cell formed by fertilization in the case of a plant.

The germ cell may be any one of a fertilized egg cell, an egg cell, and a sperm cell. A fertilized egg cell is preferable. Alternatively, the fertilized egg cell is preferably produced and obtained by isolating an egg cell and a sperm cell from a plant before pollination and fertilization and thereafter fusing them, without limitation. Alternatively, the fertilized egg cell may be a fertilized egg cell that is isolated from a tissue of a plant containing the embryo sac, i.e., a fertilized egg cell from a plant that has been pollinated and fertilized. Without limitation, it is more preferable to use a fertilized egg cell, which is a polyploid and the genome sequence of which has been determined, than to use a sperm cell and an egg cell which are haploids before fertilization, since the present invention aims at transformation by gene introduction or recombination, introduction of a substance into a plant cell for genome editing or the like, and particularly gene introduction.

In this description, an "egg cell" means a female gamete formed by meiosis of the embryo sac mother cell in the pistil. Although the method for isolating an egg cell is not limited, the ovary is cut in a solution with an appropriate osmotic pressure, and then an egg cell exposed on the cut surface can be isolated using a glass capillary under a microscope, for example.

In this description, a "sperm cell" means a male gamete formed by meiosis of a pollen mother cell in the anther of a stamen. Although the method for isolating a sperm cell is not limited, upon immersion of pollen collected from the anther in a solution with an appropriate osmotic pressure, the contents of the pollen containing a sperm cell are released from the pollen into the solution after a lapse of several minutes, and then a sperm cell can be isolated using a glass capillary under a microscope, for example.

In this description, a "fertilized egg cell" means a cell in which a sperm cell and an egg cell are fused.

In this description, the plant germ cell represents a sperm cell or an egg cell isolated from a stamen or the pistil, a fertilized egg cell in which the cells isolated are fused, or a fertilized egg cell isolated from the pistil.

### Method for obtaining fertilized egg cell

According to one embodiment of the present invention, an egg cell and a sperm cell of a plant may be fused in vitro to produce a fertilized egg cell. That is, an egg cell and a sperm cell can be first isolated from a plant to produce a fertilized egg cell in vitro by a known method such as electrofusion (also referred to as gamete fusion).

Electrofusion is a method of fusing two or more cells in vitro by electric stimulation. Specifically in this description, a fertilized egg cell can be produced by applying an electric pulse to an egg cell and a sperm cell isolated in a solution with an appropriate osmotic pressure and thereby fusing the cells.

In the case of fusing the cells by electrofusion, conditions such as voltage and distance between electrodes can be appropriately determined by those skilled in the art corresponding to the type of the plant or the sizes of the cells. For example, the conditions disclosed in Japanese Patent Laid-Open No. 2016-63785 (PTL 1) can be used.

When one fusion cell (a fertilized egg cell) is produced by electrofusion of a sperm cell and an egg cell, the lower limit of the DC voltage is preferably 10 kV or more, more preferably 11 kV or more, further preferably 12 kV or more. Further, the upper limit is preferably 17 kV or less, more preferably 16 kV or less, further preferably 15 kV or less. The upper limit and the lower limit can be appropriately selected by those skilled in the art.

Further, the lower limit of the distance between electrodes is preferably 1.5 times or more, more preferably 2 times or more, further preferably 2.5 times or more, most preferably 3 times or more of the sum of the diameters of the egg cell and the sperm cell to be fused. Further, the upper limit is preferably 6 times or less, more preferably 5 times or less, further preferably 4 times or less. The upper limit and the lower limit can be appropriately selected by those skilled in the art. Examples of the method for measuring the diameter of a cell include a method of measuring the diameter using a micrometer eyepiece mounted on a microscope, and a method of importing an image captured with a microscope into a computer and measuring the diameter using an image analysis software.

Further, the distance between electrodes can be appropriately selected by those skilled in the art. For example, the lower limit is preferably 80 µm or more, more preferably 90 µm or more, further preferably 100 µm or more. Further, the upper limit is preferably 240 µm or less, more preferably 220 µm or less, further preferably 200 µm or less. The upper limit and the lower limit can be appropriately selected by those skilled in the art.

The osmotic pressure of the solution used when one fusion cell is produced by electrofusion of a sperm cell and an egg cell can be appropriately selected corresponding to the type of the plant to be used. For example, in the case of rice, the lower limit is preferably 380 mosmol/kg H₂O or more, more preferably 390 mosmol/kg H₂O or more, further preferably 400 mosmol/kg H₂O or more. Further, the upper limit is preferably 470 mosmol/kg H₂O or less, more preferably 460 mosmol/kg H₂O or less, further preferably 450 mosmol/kg H₂O or less. In the case of maize, the lower limit is preferably 500 mosmol/kg H₂O or more, further preferably 530 mosmol/kg H₂O or more. Further, the upper limit is preferably 700 mosmol/kg H₂O or less, further preferably 680 mosmol/kg H₂O or less. The upper limit and the lower limit can be appropriately selected by those skilled in the art.

Alternatively, other known cell fusion methods such as the calcium fusion method and the PEG fusion method may be used for cell fusion of an egg cell and a sperm cell. The "calcium fusion method" utilizes a property of cell membranes that fusion of cell membranes tends to occur depending on the calcium concentration. The "PEG fusion method" utilizes cell fusion by treating a cell with polyethylene glycol (PEG) to bind cell membranes, followed by removal of PEG.

Alternatively, a fertilized egg cell may be produced in a plant by natural fertilization to obtain the fertilized egg cell produced from the plant. The method for obtaining a fertilized egg cell using natural fertilization is, for example, a method of exposing the stigma, attaching pollen for pollination, and thereafter isolating a fertilized egg cell from a tissue containing the embryo sac. The fertilized egg cell can be isolated from the plant by extracting the ovary immediately after fertilization from the plant after pollination, cutting the ovary in a solution with an appropriate osmotic pressure, and isolating the fertilized egg cell exposed on the cut surface under a microscope using a glass capillary or the like. Alternatively, the fertilized egg cell can be released to be isolated, for example, by dissecting a tissue such as nucellus using a glass needle under a microscope, after treatment of the ovary or ovules with an enzyme solution for a certain time. In this description, the natural fertilization may be artificial mating in which pollen is artificially attached to the stigma or may be natural mating.

### Substance introduction

The method of the present invention for introducing a substance into a plant comprises a "step of introducing a substance into a plant germ cell with incomplete cell wall formation".

In the present invention, the substance introduced into the plant is a substance with a size and properties which can be supplied into a cell that is a target. The substance may be naturally present or artificially produced. Examples thereof include various biomolecules and compounds. Examples of the biomolecules include nucleic acids, proteins, peptides, polysaccharides, lipids, and cell organelles. According to one embodiment, the substance is selected from the group consisting of nucleic acids, proteins, and peptides.

The nucleic acids are not specifically limited and may be RNA, DNA, and a conjugate or mixture of the two. Preferably, the nucleic acids are circular DNA like vectors, linear DNA, circular RNA, or linear RNA. Those having any length corresponding to the transformation method to be used can be used. For example, the length of nucleic acids is preferably 100 kb or less, more preferably 50 kb or less. The length is further preferably 30 kb or less, particularly preferably 20 kb or less, most preferably 10 kb or less. It may also be a complex of nucleic acids and proteins like chromosomes.

Proteins for genome editing such as nucleases including Cas9 nuclease, modifying enzymes, and antibodies can be introduced. Without limitation, the size of the proteins is a molecular weight of preferably 300 kDa or less, more preferably 200 kDa or less, further preferably 150 kDa or less.

Peptides generally refer to molecules in which various amino acids are linked in a fixed order by amide bonds (also referred to as "peptide bonds") and generally have a shorter length than proteins. Preferably, the length is 100 a.a. or less, more preferably 50 a.a. or less.

"Polysaccharides" generally refer to materials in which two or more monosaccharide molecules are polymerized by glycosidic bonds. For example, they exhibit properties different from those of monosaccharides which are constituent units, like starch. Examples thereof include starch (amylose and amylopectin), glycogen, cellulose, chitin, agarose, carrageenan, heparin, hyaluronic acid, pectin, xyloglycan, and glucomannan.

"Lipids" generally refer to substances that are isolated from organisms and are insoluble in water. They are defined by the solubility, not by specific chemical or structural properties. In the biochemical definition, they are "in-vivo or biologically-derived molecules having long-chain fatty acids or hydrocarbon chains". Examples thereof include: (i) simple lipids (such as acyl glycerol and ceramide) formed by ester bonding of only an alcohol and a fatty acid, (ii) complex lipids (such as phospholipid, sugar lipid, and lipoprotein) containing a phosphate or sugar in the molecule and generally having a skeleton of sphingosine or glycerin, and (iii) derived lipids (such as fatty acid, tempenoid, steroid, and carotenoid) that are hydrophobic compounds derived from simple lipids or complex lipids by hydrolysis.

"Cell organelles" generally refer to structures with specifically differentiated morphologies or functions inside cells and may be called intracellular organs or organelles. According to one embodiment, examples thereof include structures surrounded by biomembranes such as nuclei, endoplasmic reticula, the Golgi apparatus, endosomes, lysosomes, mitochondria, chloroplasts, and peroxisomes. Examples further include cell skeletons and structures composed of supercomplexes of non-membrane proteins such as centrosomes, flagella, and cilia. Examples can further include nucleoli and ribosomes.

The "substance" can also include metal ions, compounds, and the like other than biomolecules. Two or more types of substances such as nucleic acids, proteins, peptides, polysaccharides, lipids, metal ions, and compounds may be introduced in combination. For example, nucleic acids may be two or more types of DNAs or RNAs, or a combination of DNA and RNA. Different types of substances such as a nucleic acid and a protein may be introduced simultaneously or as a complex.

Further, the substances may be supplied while being carried by or contained in a carrier for introduction depending on the properties of substances to be introduced. Here, the carrier is a medium (vehicle) for supplying foreign substances into a cell. Examples thereof include liposomes, particles or whiskers composed of metals (such as gold and tungsten) or inorganic substances (such as silicon compounds), alginate beads, and viral substances (such as coating proteins). Further, substances that promote substance introduction into a cell may be supplied simultaneously. Examples thereof include proteins such as fibronectin, peptides, chelate compounds (such as EDTA), and inorganic microstructures (such as nanostructured silica).

The method for introducing a substance into a plant is not specifically limited, as long as it is a known method by which a desired substance can be introduced into a plant, and can be appropriately selected corresponding to the type of the plant. For example, physicochemical methods (direct DNA introduction methods) such as the polyethylene glycol method (PEG method), the electroporation method, the particle gun method, the microinjection method, and the whisker method, or biological methods (indirect DNA introduction methods) such as the Agrobacterium method can be preferably used. Direct introduction methods are preferable, and the PEG method or the electroporation method is further preferable.

The PEG method is a method of incorporating substances such as DNA into a plant cell by allowing polyethylene glycol (PEG) to affect a protoplast, but the mechanism of uptake of substances has not been well known, yet. The PEG method can be carried out according to a known protocol as disclosed, for example, in NPL 13 or the like. Without limitation, the concentration of PEG can be 10% to 30%.

The electroporation method is a method of feeding DNA in a cell suspension into a cell by applying an electric pulse to the cell suspension to make micropores through cell membrane. In the case of using a plant cell as a material, a protoplast with cell wall broken and removed is generally used. Substance introduction by the electroporation method can be carried out according to a known protocol. The conditions thereof can be appropriately selected by those skilled in the art depending on the plant material to be used, but the following conditions can be mentioned, for example.

The operation of applying an electric pulse to the cell by the electroporation method is performed within an electrode container such as an electrode cuvette. Electrodes are, for example, composed of metals such as platinum, gold, and aluminum, and the distance between electrodes can be appropriately selected by those skilled in the art, for example, from about 0.5 mm, about 1 mm, about 2 mm, about 4 mm, and about 10 mm.

The lower limit of the voltage per distance between electrodes of 1 mm can be appropriately selected by those skilled in the art, for example, from 5 V or more, 10 V or more, 20 V or more, 30 V or more, 40 V or more, and 50 V or more, and the upper limit of the voltage can be appropriately selected, for example, from 5000 V or less, 1000 V or less, 500 V or less, and 100 V or less. The voltage may be a single pulse or multiple pulses. In the case of multiple pulses, the voltage can be applied as pulses of 2 times, 3 times, 4 times, 5 times, 6 times, 7 times, 8 times, 9 times, 10 times, or 15 times, for example. Each interval between pulses is about 0.5 to about 500 msec, preferably about 5 to about 250 msec, more preferably about 10 to about 150 msec, further preferably about 40 to 120 msec. Further, after the pulses are applied multiple times at such intervals, a pulse at the same or a different voltage may be applied at 1 sec to 10 sec, 2 sec to 8 sec, or 3 sec to 5 sec. Further, in the case where a plurality of pulses are applied, the pulse intensity may be the same or different. The lower limit of the duration of each pulse may be selected from 0.01 msec or more, 0.1 msec or more, and 1 msec or more, and the upper limit thereof may be selected from 15 msec or less, 10 msec or less, 5 msec or less, and 3 msec or less.

The solution used in the electroporation method may be one used in the electroporation method that has been conventionally performed on a plant. Examples thereof include solutions containing inorganic salts such as KCl, NaCl, and CaCl₂ using PBS, HEPES, or MES as a buffer, and solutions containing organic salts such as potassium aspartate, calcium gluconate, and potassium glutamate. Further, solutions used when the electroporation method is performed on cells or tissues of animals can be used. Examples thereof include Opti-MEM medium manufactured by Thermo Fisher Scientific K.K. The osmotic pressure of a solution can be appropriately selected corresponding to the type of the plant to be used. For example, in the case of rice, the lower limit is preferably 380 mosmol/kg H₂O or more, more preferably 390 mosmol/kg H₂O or more, further preferably 400 mosmol/kg H₂O or more. Further, the upper limit is preferably 470 mosmol/kg H₂O or less, more preferably 460 mosmol/kg H₂O or less, further preferably 450 mosmol/kg H₂O or less. In the case of maize, the lower limit is preferably 500 mosmol/kg H₂O or more, further preferably 530 mosmol/kg H₂O or more. Further, the upper limit is preferably 700 mosmol/kg H₂O or less, further preferably 680 mosmol/kg H₂O or less.

The concentration of the substance to be introduced is not limited but is 1 ng/µL to 2000 ng/µL, preferably 10 ng/µL to 1000 ng/µL, more preferably 20 ng/µL to 500 ng/µL.

In the PEG method and the electroporation method, introduction or co-transformation of a plurality of types of substances can be carried out by dissolving two or more types of substances such as DNA in a suspension and adding PEG or applying an electric pulse in the presence of a plant cell.

The substance introduction may be, for example, such that nucleic acids are introduced into a cell, and the nucleic acids are incorporated into the genome and are stably held therein, or may be temporary introduction such that nucleic acids are not constantly and stably held in a cell and are held only for a certain time, like gRNA and Cas9 protein in genome editing.

### Plant germ cell with incomplete cell wall formation

In the method of the present invention for introducing a substance into a plant, a substance selected from the group consisting of nucleic acids, proteins, and peptides is introduced into the "plant germ cell with incomplete cell wall formation".

It is one of the features of the present invention that the plant germ cell isolated before substance introduction is not treated and does not need to be treated with plant tissue-degrading enzymes by utilizing the "plant germ cell with incomplete cell wall formation".

Plant tissue-degrading enzymes generally refer to enzymes that directly or indirectly act on pectin, cellulose, hemicellulose, other matrix polysaccharides, phospholipids, proteins, and the like in the periphery of tissues and cells of a plant to degrade them. Examples thereof include pectinase, cellulase, protease, hemicellulase, and mixtures thereof. However, a treatment of a plant cell with such a plant tissue-degrading enzyme may adversely affect the viability of the cell and may possibly be one of the causes for the difficulty in culturing plant tissues. Since the present invention enables culture, substance introduction, and transformation without a treatment of a plant cell with plant tissue-degrading enzymes, transformants of plants, which have been conventionally difficult to transform due to the difficulty of culture or the like and thus to which useful traits could not be given, can be stably obtained with good reproducibility.

In this description, a "treatment of an isolated plant germ cell with plant tissue-degrading enzymes" includes a treatment performed on a plant germ cell for protoplastization such as gene introduction by the PEG method or the like and desirably excludes a treatment simply for isolating the plant germ cell from a tissue containing the embryo sac such as the ovary (generally using an enzyme solution with a very low concentration). The present invention includes an embodiment of simply using an enzyme solution with a very low concentration for isolating the plant germ cell. Specifically, the present invention includes the case where, after plant tissue-degrading enzymes are added to the ovule or the nucellus to isolate an egg cell, and a sperm cell is fused with the isolated egg cell to obtain a fertilized egg cell, a substance is introduced into the isolated fertilized egg cell by the PEG method or the electroporation method, and the case where, after a treatment with plant tissue-degrading enzymes is performed only to isolate a fertilized egg cell produced within a plant by natural fertilization from the ovule or the nucellus, and the fertilized egg cell is isolated, a substance is introduced into the isolated fertilized egg cell by the PEG method or the electroporation method.

The "cell with incomplete cell wall formation" is not a cell like a plant cell, which is almost entirely surrounded by a cell wall except plasmodesmata connection but a cell not surrounded by a cell wall partially or entirely. The plant germ cell with incomplete cell wall formation includes gametes for sexual reproduction including an egg cell and a sperm cell. Alternatively, it includes a fertilized egg cell which is formed by fertilization and in which cell wall formation has not started or cell wall formation has started but has not been completed yet in an incomplete state. Preferably, it is a fertilized egg cell with incomplete cell wall formation.

Cell wall formation in a plant germ cell can be checked, for example, by a known method such as cellulose staining by Calcofluor and aniline blue staining. Calcofluor is a non-specific fluorescence dye that binds to cellulose or chitin contained in cell walls of plant cells, fungi, and the like. It is excited at 300 to 440 nm (355 nm at maximum), and the maximum fluorescence of cellulose in a 0.1-M phosphate buffer solution with a pH of 7.0 is 433 nm. Without limitation, the fluorescence brightness can be measured, for example, using a confocal laser scanning microscope, and the integrated brightness can be determined by image analysis of the fluorescence intensity.

Without limitation, the "rate of cell wall formation" can be expressed, for example, as a ratio of the brightness as compared with the brightness of the cell when cell wall formation in a plant cell has been completely finished, and the cell is entirely covered by a cell wall. Without limitation, the "incomplete cell wall formation" means that the rate of cell wall formation is preferably 80% or less, more preferably 70% or less, further preferably 65% or less, further more preferably 63% or less, particularly preferably 60% or less, particularly, further preferably 50% or less, most preferably 30% or less.

According to one embodiment of the present invention, a fertilized egg cell can be used as a plant germ cell. In such a case, the fertilized egg cell is obtained by (1-i) fusing an egg cell and a sperm cell of a plant to produce a fertilized egg cell, or (1-ii) isolating a fertilized egg cell of a plant from a tissue containing the fertilized egg cell; and thereafter (2) a substance is introduced into the fertilized egg cell obtained.

An egg cell before fertilization has a cell wall in a state different from that of a normal somatic cell, in which a complete cell wall is formed for the first time when an egg cell and a sperm cell are fused. Once the cell wall is formed, the cell wall needs to be removed for substance introduction. Therefore, the operation of substance introduction is preferably performed immediately after fertilization before the completion of cell wall formation. For example, in the case of maize and rice, cell wall formation starts about 20 minutes after fertilization (NPLs 15 and 16) and is completed in about 2 hours. Therefore, substance introduction is preferably performed within 2 hours (120 minutes). Further, also in the case of isolating a fertilized egg cell produced by natural fertilization in a plant from the ovule or the like, the operation of substance introduction is preferably performed immediately after the isolation of the fertilized egg cell.

However, in the case of producing a fertilized egg cell by natural fertilization within a plant, it takes time from when the pollen tube is attached to the stigma to actual fertilization in the plant and formation of a fertilized egg cell, unlike the case of fusion between an isolated egg cell and a sperm cell. Therefore, in such a case, it is preferable to specify the time period from pollination to fertilization and remove the fertilized egg cell after fertilization during the time period specified, as a method for obtaining a fertilized egg cell immediately after fertilization. For example, the time from pollination to fertilization can be estimated by fluorescence observation over time of the extension rate of the pollen tube of a pre-pollinated pollen using a staining solution such as aniline blue. In view of the time from pollination to fertilization (time to formation of a fertilized egg) thus estimated, the operation of substance introduction is preferably performed within the time period before the completion of cell wall formation in the fertilized egg cell.

The time to the completion of cell wall formation in a fertilized egg cell varies depending on the plant variety. In the method of the present invention, a substance is introduced into a "cell with incomplete cell wall formation", that is, a fertilized egg cell before the completion of cell wall formation in the cell. The time from the acquisition of a fertilized egg cell to the completion of substance introduction can be appropriately determined corresponding to the plant variety by those skilled in the art. For example, in the case of rice or maize, substance introduction is preferably performed within 180 minutes, preferably 150 minutes, more preferably 120 minutes, further preferably 60 minutes, more preferably 20 minutes, after a fertilized egg cell is obtained by fusing an egg cell and a sperm cell isolated, without limitation. Particularly in the case of maize, substance introduction is performed within 120 minutes, preferably 60 minutes, more preferably 20 minutes, after the acquisition of a fertilized egg cell.

Further, for example, in the case of rice or maize, substance introduction is performed within 6 hours, 5 hours, 4 hours, preferably 360 minutes, 240 minutes, 180 minutes, further preferably 120 minutes, more preferably 60 minutes, after pollination (mating) by natural fertilization, without limitation.

Alternatively, after a substance is introduced into either or both of an egg cell and a sperm cell before mating the egg cell and the sperm cell, the egg cell and the sperm cell may be fused to produce a fertilized egg cell. Preferably, the substance is introduced into the egg cell.

### Callus formation or embryo-like structure (embryonic cell mass) formation and redifferentiation

The method for introducing a substance into a plant of the present invention may further comprise: conducting callus formation or embryo-like structure (also referred to as embryonic cell mass) formation of the fertilized egg cell into which the substance has been introduced, after the step of introducing the substance; and redifferentiating the callused or embryonated tissue in a redifferentiation medium.

The callus formation or embryo-like structure formation step and the redifferentiation step are not specifically limited, and known methods for regenerating a plant from a fertilized egg cell can be used.

In the callus formation or embryo-like structure formation step, the substance-introduced fertilized egg cell obtained is cultured to form the embryo-like structure or callus. The step of inducing division of a fertilized egg cell and allowing the cell to grow so as to form the callus or embryo-like structure is not specifically limited, since the optimal conditions differ depending on plants, but is preferably the nurse culture method with feeder cells added. For example, the procedure can be as follows.

Pretreatment: A substance-introduced fertilized egg cell is put into a mannitol droplet (450 mosmol/kg H₂O), followed by washing for sterilization.

Culture in liquid medium: The substance-introduced fertilized egg cell is transferred to a medium, followed by standing overnight and culture by gentle shaking. The shaking speed is preferably 30 to 50 rpm, more preferably 35 to 45 rpm. The culture temperature is preferably 24 to 28°C, more preferably 25 to 27°C. The culture is preferably performed in the dark. At this time, feeder cells are preferably added to the medium to perform co-culture (nurse culture method). The culture period is preferably 4 to 14 days, more preferably 5 to 10 days.

Medium: Liquid MS medium (T. Murashige et al., Physiol. Plant., 15, 473 (1962)), B5 medium (O.L. Gamborg et al, Experimental Cell Research, 50, 151-158 (1968)), or N6 medium (Chu et al., Sci. Sinica, 18, 659-668 (1975)), and the like, to which auxins such as 2,4-dichlorophenoxyacetic acid and naphthaleneacetic acid are added.

Auxins such as indole-3-acetic acid, 2,4-D, and dicamba are preferably added to the medium. The concentration of auxins to be added is, for example, 0.1 to 3.0 mg/L, preferably 0.1 to 0.3 mg/L, more preferably 0.15 to 0.25 mg/L.

Feeder cells: Any known feeder cells can be used. Examples thereof include a rice cell suspension culture (Line Oc, manufactured by Riken BioResource Research Center), a maize nurse cell (Mol et al., 1993), and a non-morphogenic cell suspension (Kranz et al., 1991).

By this step, a spherical embryo-like structure with a diameter of about 50 to 200 µm is formed 4 to 14 days after the start of culture.

The redifferentiation step also can be carried out according to a known redifferentiation step. For example, it can be performed, as follows.

Culture: A spherical embryo-like structure is transferred to a medium not containing feeder cells, followed by further culture for about 10 to 14 days. Thereafter, the embryo-like structure is cultured in an arbitrary medium not containing auxins such as MS medium to form a plant. At this time, the culture is preferably performed under light irradiation, and the light is, for example, preferably 50 to 180 µmol/m² per second, more preferably 70 to 150 µmol/m² per second.

Medium: Solid media such as MS medium, B5 medium, and N6 medium, using agarose, agar, gellan gum, gelrite, or the like are mentioned, for example.

### Substance-introduced plant

The present invention further includes a substance-introduced plant obtained by the method of the present invention. Before the present invention, it has been difficult or impossible to obtain substance-introduced plants, particularly, of "difficult-to-culture" plants and varieties. The present invention enables a substance-introduced plant of such plants and varieties to be efficiently obtained by a simple method.

Further, the substance-introduced plant obtained by the method of the present invention includes not only plants with nucleic acids such as plasmids and gene sequence fragments, proteins or peptides for genome editing or the like being introduced and held within the plants, but also transformed plants obtained by introducing substances, particularly, genes, genome-edited plants by introduction of substances associated with genome editing such as Cas9 and guide RNA, and their progenies and clones.

### EXAMPLES

Hereinafter, the present invention will be described in detail based on examples, but the present invention is not limited to these examples. Those skilled in the art can easily modify and change the present invention based on the disclosure of this description, and such modifications and changes are included in the technical scope of the present invention.

### Example 1: Isolation of egg cell and sperm cell of rice

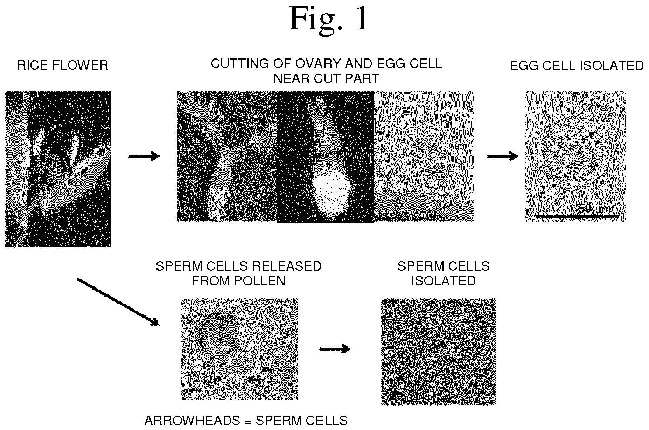

In this example, an egg cell and a sperm cell were isolated from a rice flower (Fig. 1).

The egg cell was isolated as follows. An unbloomed flower obtained from the ear of rice was dissected, to collect the ovary. The ovary was put into a 3.5-cm plastic Petri dish containing 3 mL of a 6% mannitol solution (370 mosmol/kg H₂O). The ovary with the stigma removed was submerged into 3 mL of a 6% mannitol solution (370 mosmol/kg H₂O) in a new 3.5-cm plastic Petri dish, and the lower part of the ovary was cut at the bottom of the Petri dish using a laser blade (FA-10, manufactured by FEATHER Safety Razor Co., Ltd). The egg cell released from the cut ovary was observed by microscopy, and the egg cell was isolated with a microcapillary. About 10 to 15 egg cells were obtained from 30 to 40 ovaries. Each egg cell had a diameter of 40 to 50 µm. The egg cells isolated were moved into a droplet on a cover glass using a microcapillary.

The droplet on the cover glass was created by the following method.
1) The periphery of the cover glass (20 mm × 40 mm) is immersed in a 1,1,1-trichloroethane solution containing 2% dichloromethyl silane, and then dried;
2) 0.2 to 0.3 mL of mineral oil (Embryo Culture-tested Grade, manufactured by Sigma-Aldrich Corporation, 1001279270) is placed on the center of the cover glass; and
3) 1 to 2 µL of a 6% mannitol solution (370 mosmol/kg H₂O) is inserted into the mineral oil with a micro pipette.

The sperm cell was isolated as follows. An unbloomed flower obtained from the ear of rice was disassembled, to collect the anther. The anther was put into a 3.5-cm plastic Petri dish containing 3 mL of a 6% mannitol solution (370 mosmol/kg H₂O). After the anther was submerged into the mannitol solution, the anther was dissected using tweezers to disperse pollen into the mannitol solution. After a lapse of several minutes, the pollen burst, and its contents containing the sperm cell were released in the mannitol solution. Each sperm cell had a diameter of 8 to 10 µm. The sperm cell was moved into a droplet on a cover glass using a microcapillary.

### Example 2: Production of fertilized egg cell of rice by fusion of gametes

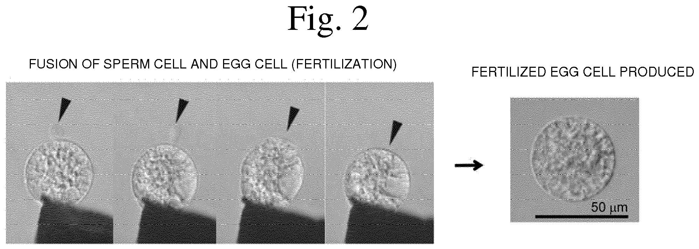

In this example, a fertilized egg cell of rice (in-vitro fertilized egg cell of rice) was produced in vitro by electrofusion of gametes (Fig. 2).

One sperm cell and one egg cell isolated by the method shown in Example 1 were moved into a droplet on a cover glass. Thereafter, these cells were aligned on electrodes (CUY5100-100Ti, Nepa Gene Co., Ltd.) under alternating current (1 MHz, 0.4 kV/cm, ECFG21, Nepa Gene Co., Ltd). A mannitol solution (520 mosmol/kg H₂O) was added into the droplet in an amount equivalent to a half to the same amount of the droplet using a microcapillary. Thereafter, a DC pulse (50 µs, 12 to 15 kV/cm, with a distance between electrodes of 50 to 150 µm) was applied to fuse gametes so as to produce a fertilized egg cell.

### Example 3: Introduction of nucleic acids into fertilized egg cell of rice

In this example, nucleic acids were introduced into the in-vitro fertilized egg cell of rice produced in Example 2.

The fertilized egg cell produced in Example 2 was treated according to this example, so that substance introduction was completed within 120 minutes after the fusion of gametes. The fertilized egg cell produced was moved into a droplet (about 2 µL) of an MMG solution (15 mM of MgCl₂, 4 mM of MES (pH 5.7), 450 mosmol/kg H₂O mannitol) and was thereafter moved into a droplet of MMG to which a plasmid (plasmid DNA for GFP expression, about 6,000 bp) with a base sequence to be introduced, 35S promoter::signal sequence::GFP::endoplasmic reticulum retention signal (HDEL)::NOS terminator, has been added. The plasmid was prepared according to the description of NPL 17. Next, the droplet containing the fertilized egg cell was mixed with a droplet (about 2 µL) of a PEG solution (obtained by adding 7.5 g of PEG4000 and 2.5 mL of 1 M calcium chloride to 12.5 mL of a mannitol solution (450 mosmol/kg H₂O) and adjusted to 25 mg using distilled water), followed by stirring 30 to 50 times using a glass capillary.

### Example 4: Culture of fertilized egg cell of rice

In this example, the fertilized egg cell of rice with nucleic acids introduced in Example 3 was cultured.

In order to culture the fertilized egg cell obtained in Example 3 with nucleic acids introduced, a medium for fertilized cell was prepared. The medium for fertilized cell was N6Z medium (Kumlehn J.et.al. (1998) Planta 205: 327-333) modified as follows: 2 g/L of CHU (N6) basal salt mixture (manufactured by Sigma-Aldrich Corporation), 0.025 mg/L of Na₂MoO₄·2H₂O, 0.025 mg/L of CoCl₂·6H₂O, 0.025 mg/L of CuSO₄·5H₂O, 0.01 mg/L of retinol, 0.01 mg/L of calciferol, 0.01 mg/L of biotin, 1 mg/L of thiamine·H₂O, 1 mg/L of nicotinic acid, 1 mg/L of pyridoxine·HCl, 1 mg/L of choline chloride, 1 mg/L of Ca-pantothenic acid, 0.2 mg/L of riboflavin, 0.2 mg/L of 2,4-D, 0.02 mg/L of cobalamin, 0.02 mg/L of p-aminobenzoic acid, 0.4 mg/L of folic acid, 2 mg/L of ascorbic acid, 40 mg/L of malic acid, 40 mg/L of citric acid, 40 mg/L of fumaric acid, 20 mg/L of Na-pyruvic acid, 1,000 mg/L of glutamine, 250 mg/L of casein hydrolyzate, and 100 mg/L of myoinositol. The osmotic pressure was adjusted to 450 mosmol/kg H₂O (pH 5.7) with glucose for production. The medium for fertilized cell (0.2 mL) produced was put into a Millicell CM insert with a diameter of 12 mm (manufactured by Millipore Corporation) and was put into a 3.5-cm plastic Petri dish containing 2 mL of the medium. Further, 40 to 60 µL of rice cell suspension culture (Line Oc, manufactured by Riken BioResource Research Center) was added to the Petri dish as feeder cells.

Using a washed and sterilized microcapillary, the fertilized egg cell with nucleic acids introduced was put into a fresh mannitol droplet (450 mosmol/kg H₂O) and thereafter was transferred onto a membrane in the CM insert containing the medium for fertilized cell.

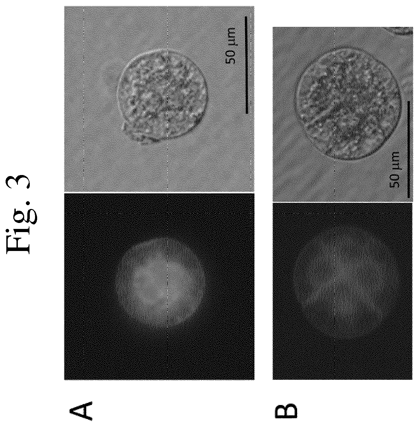

The fertilized egg cell with nucleic acids introduced was left standing in the dark at 26°C for one day, and then was observed with a fluorescence microscope, to check the expression status of the nucleic acids introduced and the cell division status based on the presence or absence of GFP fluorescence. Fig. 3A shows the results. Since GFP fluorescence was certainly observed in fertilized egg cells with nucleic acids introduced, introduction of nucleic acids could be confirmed. Further, as a result of observing the fertilized egg cells after shaking culture for 2 days, the division into early embryos was confirmed, and further light emission by GFP could be also observed in the early embryonic cells (Fig. 3B). It could be confirmed from this that the nucleic acids introduced into the fertilized egg cells were held also in the cell group of embryonic cell mass.

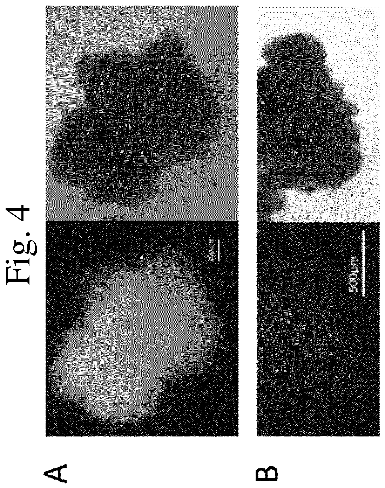

These early embryos were further cultured with shaking in the dark for about 16 days, to obtain an embryonic cell mass. At this time, the feeder cells were removed from the culture liquid 5 to 7 days after the start of culture. As a result of observing the embryonic cell mass cultured for 18 days, a cell mass from which light emission by GFP was detected (Fig. 4A) and a cell mass in which fluorescence was not detected (Fig. 4B) were observed. This revealed that there were two cases, in which GFP was continuously expressed from the fertilized egg stage to the cell mass stage, and in which GFP was transiently expressed from the fertilized egg stage to the early embryo stage. Further, since fluorescence was observed in an embryonic cell mass that had been continuously cultured for one week or more, it was confirmed that nucleic acids introduced into the fertilized egg transformed to stably produce fluorescent proteins. The above results revealed that a gene can be introduced into a plant to be transiently or stably expressed by the method of the present invention.

### Example 5: Differentiation induced by culture of rice embryonic cell mass

In this example, differentiation was induced by culture of the rice embryonic cell mass obtained in Example 4.

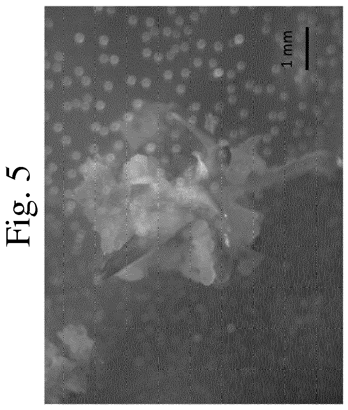

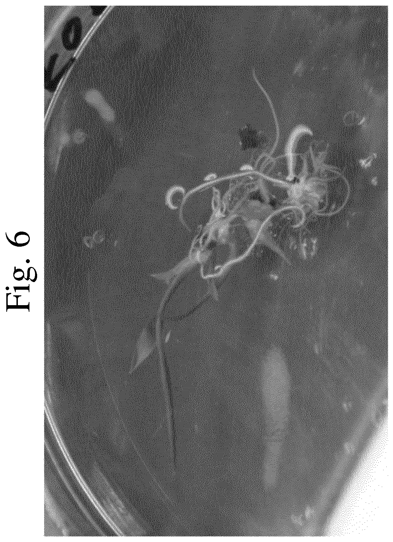

The rice embryonic cell mass obtained in Example 4 was transferred into differentiation-inducing medium 1 (modified MS medium; MS salt, MS vitamin, 100 mg/L of myoinositol, 2 g/L of casamino acid, 30 g/L of sucrose, 30 g/L of sorbitol, 0.2 mg/L of 1-naphthaleneacetic acid (NAA), 1 mg/L of kinetin, and 0.3% of gelrite). The culture was performed at 30°C under continuous photoirradiation. Shoots differentiated 10 days after transplantation (Fig. 5). Further, the embryonic cell mass in which shoots differentiated was transferred into differentiation-inducing medium 2 (modified MS medium; MS salt, MS vitamin, 100 mg/L of myoinositol, 30 g/L of sucrose, and 0.3% of gelrite). The culture was performed at 30°C under continuous photoirradiation. Seedlings were obtained 7 days after transplantation (Fig. 6). The seedlings were redifferentiated according to the description of NPL 18.

### Example 6: Relationship between time after fusion and substance introduction efficiency

In this example, the stage of a fertilized egg in which a substance could be introduced by the PEG method without an enzymatic treatment was investigated. The time elapsed after electrofusion was used as an indicator of the stage of the fertilized eggs. The plasmid DNA for GFP expression used in Example 3 was introduced after a lapse of a certain time into a fertilized egg cell of rice produced using electrofusion according to Examples 1 and 2, and the introduction of nucleic acids was confirmed as in Example 4. Thereafter, the fertilized egg cell in which the introduction of nucleic acids had been confirmed was continuously cultured under the conditions of Example 4, and the division into early embryos was confirmed. Table 1 shows the results. From the results, no difference in introduction efficiency was recognized between substance introduction 5 to 20 minutes after the fusion of gametes and substance introduction 120 minutes after the fusion of gametes. Since there was no difference in substance introduction efficiency 5 to 20 minutes and 120 minutes after the fusion of gametes, it is understood that the substance introduction was not caused by the influence of electrofusion in producing the fertilized egg cell but by the PEG method. After a lapse of 240 minutes, the introduction of nucleic acids was confirmed with a low efficiency, but the fertilized egg did not divide.

**[Table 1]**

| Elapsed time (min) after fusion of gametes | The number of fertilized eggs | The number of fertilized eggs with introduction confirmed | The number of fertilized eggs with division confirmed |
|---|---|---|---|
| 5-20 | 34 | 16 | 15 |
| 60 | 7 | 4 | 3 |
| 120 | 18 | 8 | 8 |
| 240 | 6 | 1 | 0 |

### Example 7: Measurement of degree of cell wall formation

In this example, the degree of cell wall formation in a fertilized egg cell of rice was measured.

As described in Example 2, 0.005% Calcofluor staining was performed on a fertilized egg cell after a lapse of 2 hours from electrofusion and a fertilized egg cell 20 hours after electrical induction that was estimated to have a sufficiently developed cell wall, and the fluorescence brightness was measured using a confocal laser scanning microscope. As a result of image analysis of three cells for each to determine the integrated brightness and comparison thereof, it was confirmed that the fluorescence brightness by Calcofluor detected from cell walls 2 hours after fertilization was about 63% of that of the fertilized egg cells after 20 hours.

### Example 8: Estimation of relative amount of plasmid in fertilized egg cell of rice with nucleic acids introduced

In this example, plasmid nucleic acids were introduced into a fertilized egg cell of rice, and the relative amount of the plasmid in the cell was estimated.

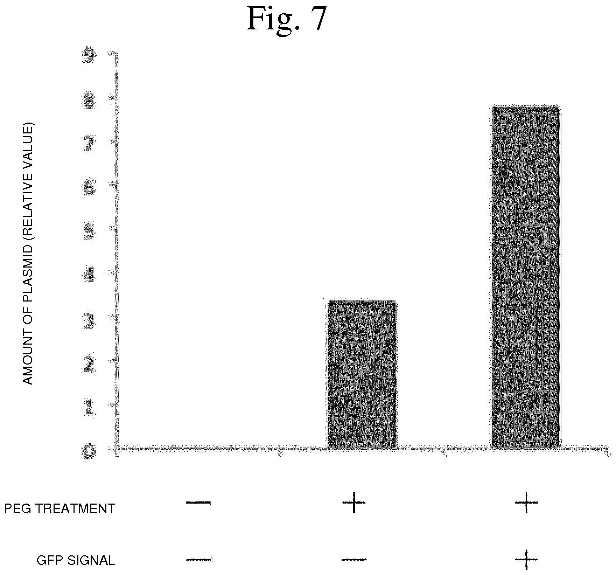

According to Examples 1 to 3, a fertilized egg cell of rice was produced by electrofusion of gametes, and nucleic acids were introduced thereinto by the PEG method. However, the process was performed 10 minutes to 1 hour after the electrofusion using a plasmid (plasmid DNA for GFP expression, about 6,000 bp) DNA containing 35S promoter::signal sequence::GFP::endoplasmic reticulum retention signal (HDEL)::NOS terminator, so that the substance introduction was completed within 120 minutes from the fusion. Thereafter, the fertilized egg cell was cultured according to Example 4, and the fluorescence derived from GFP was observed using a fluorescence microscope. Thereafter, fertilized egg cells were each transferred into 10 µL of a quantitative PCR solution one by one using a microcapillary. At this time, LightCycler 480 SYBR Green I Master (Roche Applied Science) as the quantitative PCR solution and specific primers configured to amplify SP-GFP-ER in the plasmid (5'-TCTAGAATGGTGAGCAAGGGCGAG-3' and 5'-TGGTGCAGATGAACTTCAGG-3') as primers were used. The relative amount of the plasmid in the fertilized egg cell was estimated with a PCR reaction cycle of 94°C for 10 seconds, 55°C for 10 seconds, and 72°C for 10 seconds, using a fertilized egg cell without gene introduction as the control. Fig. 7 shows the results.

As shown in Fig. 7, it was confirmed that nucleic acids were introduced not only into cells in which fluorescence derived from GFP was observed with the fluorescence microscope, but also into some individuals among the cells in which fluorescence was not observed. Further, there was a positive correlation between the GFP fluorescence intensity and the amount of the plasmid introduced, in individuals in which fluorescence derived from GFP was observed.

### Example 9: Isolation of egg cell and sperm cell of maize

In this example, an egg cell and a sperm cell of maize were isolated. The egg cell and the sperm cell were isolated with reference to Kranz E. and Loerz H. (NPL 1) as follows.

The ear of maize (variety: A188) in a mating period grown in a greenhouse and bagged before blooming was tested. A nucellus slice containing the embryo sac was released from the ovule of the ear collected, was added to 1.5 mL of an enzyme solution in a 3.5-cm plastic Petri dish, and was left standing at room temperature. The enzyme solution used was obtained by adding 0.33% cellulase (manufactured by Worthington Biochemical Corporation), 0.1% Macerozyme R10 (manufactured by Yakult Honsha Co., Ltd.), and 0.017% Pectolyase Y23 (manufactured by Morishin Pharmaceutical Co., Ltd.) to a 10% mannitol solution (650 mosmol/kg H₂O).

After enzymatic treatment for 20 to 30 minutes, an egg cell was isolated using two glass needles. The nucellus slice was fixed with one of the glass needles so as not to move, and tissues in the area in which the fertilized egg cell was estimated to be present were scraped out with the other glass needle, thereby isolating the egg cell. The area in which the egg cell was located was estimated based on the position of the embryo sac observed in the nucellus slice. The egg cell isolated was moved into a mannitol droplet on a cover glass using a glass capillary. The mannitol droplet on the cover glass was produced according to Example 1, and the operation was performed so that the enzyme solution from which the egg cell was isolated did not incorporate into the mannitol droplet as much as possible.

The sperm cell was isolated as follows. Pollen was collected from the tassel of maize (variety: B73) into a 3.5-cm plastic Petri dish, and 3 mL of a 10% mannitol solution (650 mosmol/kg H₂O) was added thereto. After about 3 to 5 minutes, the pollen burst in the mannitol solution, pollen contents containing a sperm cell were released in the mannitol solution. Each sperm cell had a diameter of 10 µm. The sperm cell was moved into a mannitol droplet on a cover glass using a glass capillary.

### Example 10: Production of fertilized maize egg by fusion of gametes

In this example, a fertilized egg cell of maize was produced in vitro by fusing gametes by electrofusion.

One sperm cell and one egg cell isolated by the method shown in Examples 8 and 9 were moved into a droplet on a cover glass. Thereafter, these cells were aligned on electrodes (CUY5100-100Ti, Nepa Gene Co., Ltd.) under alternating current (1 MHz, 0.4 kV/cm, ECFG21, Nepa Gene Co., Ltd). Thereafter, a DC pulse (50 µs, 12 to 15 kV/cm, with a distance between electrodes of 50 to 150 µm) was applied to fuse male and female gametes and produce a fertilized egg cell.

### Example 11: Introduction of nucleic acids into fertilized maize egg

In this example, nucleic acids were introduced into the in-vitro fertilized egg cell of maize produced in Example 10.

The fertilized egg cell produced in Example 10 was treated according to this example so that substance introduction was completed within 30 to 90 minutes after the fusion of gametes. The fertilized egg cell produced was moved into a droplet (about 2 µL) of an MMG solution (15 mM of MgCl₂, 4 mM of MES (pH 5.7), and 650 mosmol/kg H₂O mannitol) and thereafter was further moved into a droplet of MMG for introducing nucleic acids, to which a plasmid (plasmid DNA for GFP expression, about 6,000 bp) with a base sequence to be introduced, 35S promoter::signal sequence::GFP::endoplasmic reticulum retention signal (HDEL)::NOS terminator, has been added at the concentration of 160 ng/µL. The plasmid was prepared according to the description of NPL 17. Next, the droplet for introducing nucleic acids containing the fertilized egg cell was mixed with a droplet (about 2 µL) of a PEG solution (obtained by adding 7.5 g of PEG4000 and 2.5 mL of 1 M calcium chloride to 12.5 mL of a mannitol solution (650 mosmol/kg H₂O) and adjusted to 25 mg using distilled water), followed by stirring 30 to 50 times using a glass capillary.

Using a washed and sterilized microcapillary, the fertilized egg cell with nucleic acids introduced was put into a fresh 10% mannitol droplet (650 mosmol/kg H₂O) and thereafter was transferred onto a membrane in the CM insert containing the medium for fertilized cell.

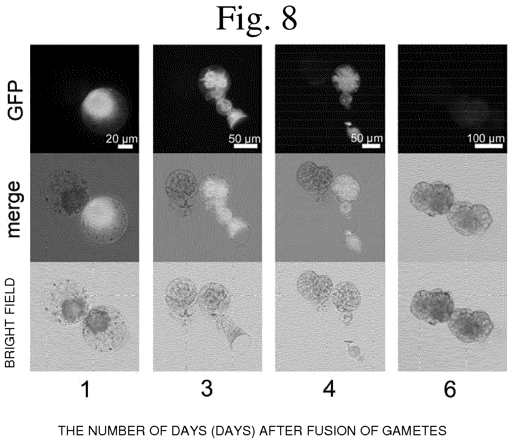

The fertilized egg cell with nucleic acids introduced was allowed to stand still in the dark at 26°C for one day, followed by shaking culture continuously. The cell after culture was observed with an inverted fluorescence microscope, to check the expression status of the nucleic acids introduced and the cell division status based on the presence or absence of GFP fluorescence. The light emission by GFP was certainly observed in the fertilized egg cell with nucleic acids introduced, and thus introduction of nucleic acids could be confirmed. Further, as a result of observing the fertilized egg cell with nucleic acids introduced after shaking culture for 6 days, division into early embryos was confirmed, and further light emission by GFP in early embryonic cells could be also observed (Fig. 8). It could be confirmed from this that the nucleic acids introduced into the fertilized egg cell were stably held also in the cell group of the embryonic cell mass of maize.

The medium for fertilized cell was produced as follows.

NH₄NO₃ in the MS medium was modified to 165 mg/L, and the organic matter composition was as follows: 1 mg/L of nicotinic acid, 10 mg/L of thiamine·HCl, 1 mg/L of pyridoxine·HCl, 0.025 mg/L, 750 mg/L of glutamine, 150 mg/L of proline, 100 mg/L of asparagine, and 100 mg/L of myoinositol. 2 mg/L of 2,4-D was added thereto, and the osmotic pressure was adjusted with glucose to 650 mosmol/kg H₂O (pH 5.7) for production. The medium for fertilized cell produced was put into a Millicell CM insert with a diameter of 12 mm (manufactured by Millipore Corporation) and was put into a 3.5-cm plastic Petri dish containing 2 mL of the medium. Further, 40 to 60 µL of rice cell suspension culture (Line Oc, manufactured by Riken BioResource Research Center) was added to the Petri dish as feeder cells to give the medium for fertilized cell.

### Example 12: Measurement of degree of cell wall formation in fertilized egg cell of rice obtained by natural fertilization

In this example, the degree of cell wall formation in the fertilized egg cell obtained by natural fertilization (artificial mating) was measured.

A rice variety, Yukihikari, cultivated in an environment of a temperature range at a day temperature of about 28°C and a night temperature of about 23°C and a humidity of 40 to 90% was artificially mated. The artificial mating was performed by pinching florets of rice immediately before blooming with fingers. It was cultivated in the same environment for 90 minutes until the isolation operation was started. A fertilized egg cell was isolated by the same operation as in the isolation of an egg cell according to Example 2. The cell wall in the fertilized egg 3 hours, 5 hours, 8.5 hours, and 20 hours after the artificial mating was stained by 0.005% Calcofluor. Meanwhile, the fertilized egg cell was handled in a temperature range at about 23°C. The fertilized egg cell stained by Calcofluor for 10 minutes was imaged with a microscope, AxioObserver A1, manufactured by Carl Zeiss AG provided with a filter, CFW-LP01-Clinical. The imaging conditions with an exposure time of 25 msec and a white balance of 3200 K were used. The fluorescence brightness by Calcofluor per cell was measured using an image analysis software ZEN2. With the fluorescence brightness of the fertilized egg 20 hours after mating, in which the cell wall was estimated to be sufficiently developed, taken as 100%, the proportion of the fluorescence brightness of the fertilized egg several hours after mating was taken as the degree of cell wall formation. Table 2 shows the results. The degree of cell wall formation in the fertilized egg cell was 38% for 3 hours after mating, 52% for 5 hours after mating, and 84% for 8.5 hours after mating.

**[Table 2]**

| Time (h) after mating | Fluorescence brightness per cell | Proportion (%) of fluorescence brightness with respect to fertilized egg 20 hours after mating |
|---|---|---|
| 3 | 427 | 38 |
| 5 | 582 | 52 |
| 8.5 | 944 | 84 |
| 20 | 1121 | 100 |

### Example 13: Introduction of nucleic acids into fertilized egg cell of rice obtained by natural fertilization by electroporation method

In this example, GFP nucleic acids were introduced by electroporation into a fertilized egg cell of rice obtained by artificial mating.

Rice was artificially mated according to Example 12 to isolate a fertilized egg cell. A linear DNA fragment composed of a base sequence encoding maize ubiquitin promoter::maize ubiquitin intron::GFP::NOS terminator was introduced into the isolated fertilized egg cell so that the time to the completion of the DNA fragment introduction was 4 hours after mating. An Opti-MEM medium containing the DNA fragment at a concentration of 100 ng/µL with an osmotic pressure adjusted to 450 mOsmol/kg H₂O using mannitol was mixed with the fertilized egg cell. Using NEPA21, manufactured by Nepa Gene Co., Ltd., electroporation was performed. As the conditions for electroporation, 1-mm width electrodes were used, and a voltage of 30 V was applied 4 times at a pulse width of 2.0 msec and a pulse interval of 50 msec.

The fertilized egg cell with nucleic acids introduced by the electroporation method was cultured according to the method of Example 4. GFP introduction in a callus formed from the embryonic cell mass was confirmed by the PCR method and the sequence of the amplified fragment on the 42nd day of culture. At this time, specific primers (5'-atggtgagcaagggcgag-3' and 5'-ccatgatatagacgttgtggctg-3') configured to amplify GFP were used as PCR primers. As a result of a sequence analysis of the DNA fragment obtained by the PCR reaction, the base sequence of the PCR product matched the GFP sequence.

This result showed that the DNA fragment (GFP) was certainly introduced into the fertilized egg cell with nucleic acids introduced by the electroporation method. A callus derived from the fertilized egg into which the DNA fragment was confirmed to be introduced was cultured according to the method of Example 5, and differentiation was induced. As a result, redifferentiated individuals could be obtained.

### Example 14: Introduction of nucleic acids and proteins into fertilized egg cell of rice

In this example, nucleic acids and proteins were introduced into a fertilized rice egg. Specifically, using a fertilized egg cell obtained by fusing an egg cell derived from a rice into which nucleic acids encoding DsRed2 (Clontech) were introduced and a sperm cell derived from a wild rice, genome editing experiments were conducted.

The rice into which a gene encoding DsRed2 was introduced was produced as follows. A pLC41 plasmid (Genebank accession No. LC215698) containing maize ubiquitin promoter::gene sequence encoding DsRed2::NOS terminator::CaMV35S terminator, and a phosphinothricin resistance marker gene was prepared to be transformed into a rice (variety: Yukihikari) according to Hiei and Komari (NPL 18), so that a DsRed2-transformed rice was produced.

According to Examples 1 and 2, an egg cell was isolated from the DsRed2-transformed rice produced, and a sperm cell was isolated from a wild rice (variety: Yukihikari), to obtain a fertilized egg cell by electrofusion. According to Example 3, a plasmid DNA or a Cas9 protein complex was introduced into the fertilized egg cell obtained. The plasmid DNA and Cas9 protein complex to be introduced were produced as follows.

### Production of plasmid DNA to be introduced:

Using primer sets shown in Table 3, tRNA1-tRNA-gRNA2 was amplified to produce a tRNA-gRNA unit.

**[Table 3]**

| Primer | Target | Forward Primar (5' - 3') | Reverse Primar (5' - 3') |
|---|---|---|---|
| All-in-one CRISPR/Cas9 vector | gDsRed2 | TGCAGTGAAGCTGAAGGTGACCAA | AAACTTGGTCACCTTCAGCTTCAC |
| sgRNA synthesis | sgDsRed2 | TAATACGACTCACTATAGGTGAAGCTGA AGGTGACCAA | TTCTAGCTCTAAAACTTGGTCACCTTCA GCTTCAC |
| Genome PCR sequence | DsRed2-1 | ATGGCCTCCTCCGAGAACGTC | CTACAGGAACAGGTGGTGGCG |
| | DsRed2-2 | ACGTCATCACCGAGTTCATGC | GGAAGGACAGCTTCTTGTAGTCG |

The tRNA-gRNA unit produced was introduced into the BsaI site of a multiplex CRISPR/Cas9 vector using the Golden Gate cloning method, and thereby All-in-one CRISPR/Cas9 vector plasmid containing rice U6snRNA promoter::tRNA-gRNA unit was produced.

### Production of Cas9 protein complex to be introduced:

Using a GeneArt Precision gRNA Synthesis Kit (Thermo Fisher Scientific K.K.), sgRNA was synthesized. Table 3 shows the primer sets used. A Cas9 protein complex was obtained by adding 2 to 3 µg of sgRNA, 1 µg of Cas9 protein (GeneArt Platinum Cas9 having a signal tag localizing nucleic acids; Thermo Fisher Scientific K.K.) to 1.5 to 2.1 µL of a Cas9 protein storage buffer (10 mM of TrisHCl (pH 8.0), 150 mM of NaCl, 0.6 mM of TCEP, and 50% of glycerol), followed by incubation.

### Introduction of plasmid DNA or Cas9 protein:

The Cas9 protein complex obtained or the plasmid DNA adjusted to 80 to 320 ng/µL was each added to 10 µL of MMG on a cover glass and was introduced into a fertilized egg cell of rice according to Example 3. The fertilized egg with the plasmid DNA introduced or the fertilized egg with the Cas9 protein complex introduced thus obtained was cultured according to Example 4, and the fluorescence of DsRed2 was observed with a fluorescence microscope. Fig. 9 shows fluorescence micrographs and optical micrographs of the fertilized egg with the plasmid DNA introduced. It was confirmed from the results that the fluorescence of DsRed2 was quenched with culture in both of the fertilized eggs with the plasmid DNA and the Cas9 protein complex introduced.

In the fertilized egg in which the plasmid DNA was not introduced (Fig. 9A), DsRed2 expression was recognized in all the cells from immediately after the fusion of gametes (Fig. 9A: Day 0) to the cell group of the embryonic cell mass (Fig. 9A: Day 7). In contrast, in the fertilized egg with the plasmid DNA introduced (Fig. 9B), the DsRed2 emission recognized immediately after the fusion of gametes and the introduction of nucleic acids (Fig. 9B: Day 0) became not recognized as the number of culture days increased (Fig. 9B: Day 7). This was because the gene sequence encoding DsRed2 in the genome of the transformed rice was edited, as the plasmid DNA was introduced, and finally was quenched.

Further, differentiation of the obtained fertilized egg cell with the plasmid DNA introduced was induced according to Example 5, to obtain redifferentiated individuals. DNA was extracted from a leaf of a redifferentiated individual obtained, and the gene sequence encoding DsRed2 was checked using the PCR method and sequence analysis. Deletion or insertion of nucleic acids was certainly recognized in part of the gene sequence of the redifferentiated individual encoding DsRed2, indicating that the genome edition was induced.

Since the same results were obtained also in the fertilized egg cell with the Cas9 protein complex introduced, it was shown that the DsRed2 gene sequence of the transformed rice was genome-edited in the same manner also by introducing the Cas9 protein complex.

From the results, it turned out that the method of the present invention enables not only a nucleic acid plasmid but also a substance such as Cas9 protein to be introduced into a fertilized egg cell, and the method of the present invention further enables genome editing.

### Example 15: Simultaneous introduction of a plurality of types of nucleic acids into rice egg cell

A rice egg cell was isolated according to Example 1. The egg cell isolated was moved into a droplet (about 2 µL) of an MMG solution, and it was thereafter moved into a droplet of MMG to which a plasmid with a base sequence to be introduced, 35S promoter::signal sequence::GFP::endoplasmic reticulum retention signal (HDEL)::NOS terminator (NPL 17) and a plasmid containing ubiquitin promoter::ubiquitin intron::gene sequence encoding DsRed2::NOS terminator have been added. Then, according to Example 3, the droplet containing the egg cell and the two types of plasmids, and a droplet (about 2 µL) of a PEG solution were mixed, followed by stirring 30 to 50 times using a glass capillary, so that nucleic acids were introduced into the egg cell by the PEG method.

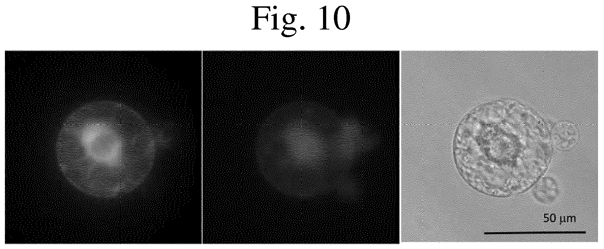

The egg cell with nucleic acids introduced was observed with a fluorescence microscope 12 to 16 hours after the introduction, to check the expression status of the nucleic acids introduced based on the presence or absence of fluorescence by GFP and DsRed2. Fig. 10 shows the results. Since light emission by both of GFP (Fig. 10: left) and DsRed2 (Fig. 10: center) was observed in the same egg cell, it could be confirmed that the two types of nucleic acids could be simultaneously introduced also into an egg cell by the method of the present invention.

### INDUSTRIAL APPLICABILITY

The present invention enables substance introduction, transformation, and culture without treating a cell with plant tissue-degrading enzymes. This enables transformants or genome-edited individuals of plants, which have been conventionally difficult to transform due to the difficulty of culture or the like and thus to which useful traits could not be given, to be easily and stably obtained with good reproducibility.

## Claims

1. A method for introducing a substance into a plant, comprising introducing a substance into a plant germ cell with incomplete cell wall formation.

2. The method according to claim 1, wherein the plant germ cell isolated is not treated with a plant tissue-degrading enzyme before introduction of the substance.

3. The method according to claim 1 or 2, wherein the rate of cell wall formation is 65% or less.

4. The method according to claim 1, wherein the plant germ cell is a fertilized egg cell.

5. The method according to any one of claims 1 to 4, comprising:
obtaining a fertilized egg cell by
(1-i) fusing an egg cell and a sperm cell of a plant to produce a fertilized egg cell, or
(1-ii) isolating a fertilized egg cell of a plant from a tissue containing the fertilized egg cell; and
(2) introducing the substance into the fertilized egg cell obtained.

6. The method according to claim 5, wherein the egg cell and the sperm cell are fused in (1-i) by electrofusion.

7. The method according to claim 5 or 6, wherein step (2) of introducing the substance is performed within 120 minutes after the fertilized egg cell is obtained.

8. The method according to claim 5 or 6, wherein step (2) of introducing the substance is performed within 60 minutes after the fertilized egg cell is obtained.

9. The method according to claim 5, wherein the egg cell and the sperm cell are fused in (1-i) by natural fertilization.

10. The method according to claim 5 or 9, wherein step (2) of introducing the substance is performed within 360 minutes after the fertilized egg cell is obtained.

11. The method according to claim 5 or 9, wherein step (2) of introducing the substance is performed within 240 minutes after the fertilized egg cell is obtained.

12. The method according to any one of claims 1 to 4, comprising:
(1) introducing the substance into either or both of the egg cell and the sperm cell of the plant; and
(2) fusing the egg cell and the sperm cell that have undergone step (1) to produce a fertilized egg cell.

13. The method according to claim 12, wherein the egg cell and the sperm cell are fused in (1) by electrofusion or natural fertilization.

14. The method according to any one of claims 1 to 13, wherein the substance is introduced using a PEG method or an electroporation method.

15. The method according to any one of claims 1 to 14, wherein the plant is a monocotyledonous plant.

16. The method according to claim 15, wherein the plant is selected from the group consisting of maize, wheat, barley, rice, and sorghum.

17. A substance-introduced plant obtained by the method according to any one of claims 1 to 16.


SEQUENCE LISTING

<110>  JAPAN TOBACCO INC.
       RIKEN
       Tokyo Metropolitan University

<120>  Method for introducing substance into plant

<130>  FA0392-17214

<150>  2017-015371
<151>  2017-01-31

<160>  12

<170>  PatentIn version 3.5

<210>  1
<211>  24
<212>  DNA
<213>  Artificial Sequence

<220>
<223>  SP-GFP-ER forward primer

<400>  1
tctagaatgg tgagcaaggg cgag                                              24


<210>  2
<211>  20
<212>  DNA
<213>  Artificial Sequence

<220>
<223>  SP-GFP-ER reverse primer

<400>  2
tggtgcagat gaacttcagg                                                   20


<210>  3
<211>  18
<212>  DNA
<213>  Artificial Sequence

<220>
<223>  GFP forward primer

<400>  3
atggtgagca agggcgag                                                     18


<210>  4
<211>  23
<212>  DNA
<213>  Artificial Sequence

<220>
<223>  GFP reverse primer

<400>  4
ccatgatata gacgttgtgg ctg                                               23

<210>  5
<211>  24
<212>  DNA
<213>  Artificial Sequence

<220>
<223>  All in one CRISPR/Cas9 vector (target gDsRed2) forward primer

<400>  5
tgcagtgaag ctgaaggtga ccaa                                              24


<210>  6
<211>  24
<212>  DNA
<213>  Artificial Sequence

<220>
<223>  All in one CRISPR/Cas9 vector (target gDsRed2) reverse primer

<400>  6
aaacttggtc accttcagct tcac                                              24


<210>  7
<211>  38
<212>  DNA
<213>  Artificial Sequence

<220>
<223>  sg RNA synthesis (target sgDsRed2) forward primer

<400>  7
taatacgact cactataggt gaagctgaag gtgaccaa                               38


<210>  8
<211>  35
<212>  DNA
<213>  Artificial Sequence

<220>
<223>  sg RNA synthesis (target sgDsRed2) reverse primer

<400>  8
ttctagctct aaaacttggt caccttcagc ttcac                                  35


<210>  9
<211>  21
<212>  DNA
<213>  Artificial Sequence

<220>
<223>  Genomic PCR sequence (target DsRed2-1) forward primer

<400>  9
atggcctcct ccgagaacgt c                                                 21


<210>  10

<211>  21
<212>  DNA
<213>  Artificial Sequence

<220>
<223>  Genomic PCR sequence (target DsRed2-1) reverse primer

<400>  10
ctacaggaac aggtggtggc g                                                21


<210>  11
<211>  21
<212>  DNA
<213>  Artificial Sequence

<220>
<223>  Genomic PCR sequence (target DsRed2-2) forward primer

<400>  11
acgtcatcac cgagttcatg c                                                21


<210>  12
<211>  23
<212>  DNA
<213>  Artificial Sequence

<220>
<223>  Genomic PCR sequence (target DsRed2-2) reverse primer

<400>  12
ggaaggacag cttcttgtag tcg                                              23